# EUROPÄISCHE PATENTSCHRIFT

(11) **EP 3 426 226 B1**
(45) Veröffentlichungstag und Bekanntmachung des Hinweises auf die Patenterteilung: **04.05.2022**
(21) Anmeldenummer: 17709641.9
(22) Anmeldetag: 07.03.2017
(51) Int. Cl.: A61K 9/08, A61K 31/505, A61K 31/728, A61P 27/04

(54) **OPHTHALMOLOGISCHE ZUSAMMENSETZUNG**
OPHTHALMOLOGIC COMPOSITION
COMPOSITION OPHTHALMOLOGIQUE

(30) Priorität: 07.03.2016 DE 102016203696
(43) Veröffentlichungstag der Anmeldung: 16.01.2019
(73) Patentinhaber: URSAPHARM Arzneimittel GmbH, 66129 Saarbrücken (DE)
(72) Erfinder: MAHLER, Markus, 66333 Völklingen (DE); WARDA, Isabella, 66111 Saarbrücken St. Johann (DE); STEINFELD, Ute, 66386 St. Ingbert (DE); HOLZER, Frank, 66386 St. Ingbert (DE)
(74) Vertreter: Pfenning, Meinig & Partner mbB
(86) Internationale Anmeldenummer: PCT/EP2017/055338
(87) Internationale Veröffentlichungsnummer: WO 2017/153415

(56) Entgegenhaltungen:
- WO-A1-03/049747
- WO-A1-03/053452
- DE-A1-102014 007 423
- US-A1- 2005 089 500

## Beschreibung

Die vorliegende Erfindung betrifft eine ophthalmologische Zusammensetzung mit hoher Viskosität. Die Zusammensetzung gemäß der Erfindung enthält oder besteht dabei aus Hyaluronsäure bzw. einem Hyaluronsäurederivat, wie beispielsweise einem ophthalmologisch akzeptablen Salz der Hyaluronsäure sowie Ectoin bzw. einem ophthalmologisch akzeptablen Ectoinderivat. Die Zusammensetzung ist weiter dadurch gekennzeichnet, dass sie keinen weiteren pharmazeutisch wirksamen Inhaltsstoff umfasst.

Brennende, juckende und tränende Augen, das Gefühl Sand in den Augen oder trockene Augen zu haben, sind Symptome eines gereizten Auges. Häufig sind dies Zeichen dafür, dass das Auge nicht ausreichend mit Feuchtigkeit versorgt ist. Eine andere Ursache können aber auch Überempfindlichkeiten gegenüber bestimmten Stoffen (z.B. Pollen, Tierhaare oder Hausstaub), sogenannte Allergien sein. Da eine Allergie mit den gleichen Beschwerden einhergeht, ist die Unterscheidung zwischen trockenen und allergisch bedingt gereizten Augen schwierig.

Bereits bekannte Lösungen zur Bekämpfung gereizter Augen richten sich in der Regel nur gegen eine der genannten Ursachen der Augenreizung bzw. Augenentzündung, wirken also entweder gegen Trockenheits-bedingte oder allergisch bedingte Reizungen und Entzündungen des Auges.

Ectoin ist ein natürlicher Stoff, der aus Mikroorganismen gewonnen wird, die in extremen Umgebungen (z.B. Salzseen) leben. Diese Mikroorganismen bilden den Naturstoff Ectoin, um sich vor den dort herrschenden extremen Umweltfaktoren zu schützen.

Aus der EP 0 671161 B1 ist bekannt, dass Ectoin und seine Derivate als Feuchtigkeitsspender in Kosmetikprodukten zur Erhöhung des Feuchtigkeitsgehaltes der Haut eingesetzt werden können. Die EP 2 214 658 B1 beschreibt die Verwendung von Ectoin in osmolythaltigen Zubereitungen zur Anwendung bei trockenen Nasenschleimhäuten. Eine Verwendung von Ectoin in Lösungen zur Vorbeugung und Behandlung einer Reizung und/oder Entzündung des Auges wird in der EP 0 671161 B1 nicht beschrieben.

Die DE 10 2014 007 423 A1 offenbart hingegen Zusammensetzungen zur Behandlung von Entzündungen am Auge, die Ectoin enthalten. Bei einer stichprobenartigen Untersuchung von 59 Patienten wird gezeigt, dass die Behandlung von Kerotoconjunctivitis sicca mit einer Zusammensetzung, die Ectoin und/oder Hydroxyectoin und/oder entsprechende Derivate dieser Stoffe umfasst, etwas effektiver ist als die Behandlung mit einer Hyaluronsäure-Lösung. Es ist nunmehr Aufgabe der vorliegenden Erfindung eine Lösung zur Vorbeugung und Behandlung bzw. zur Verwendung in einer Vorbeugung und Behandlung einer Reizung und/oder Entzündung des Auges anzugeben, wobei die Lösung sowohl zur Vorbeugung und Behandlung einer Trockenheits-bedingten als auch einer allergisch bedingten Reizung und/oder Entzündung des Auges geeignet sein soll, wodurch eine Unterscheidung der verschiedenen Ursachen der Symptome nicht mehr notwendig ist. Zudem soll die Lösung keine Bestandteile enthalten, die in der Regel zu einer Reizung des Auges oder zu einer Verschlechterung des Sehvermögens führen. Die Lösung soll dabei eine möglichst hohe Viskosität aufweisen, um zu gewährleisten, dass die Zusammensetzung gut und lange andauernd auf der Oberfläche des Auges haften bleibt. Außerdem soll die Lösung eine möglichst hohe Wasserbindungskapazität besitzen, die für eine nachhaltige Befeuchtung des Auges sorgt.

Ausgehend von dem Stand der Technik ist es ebenso Aufgabe der vorliegenden Erfindung, eine ectoinhaltige ophthalmologische Zusammensetzung anzugeben, die eine verbesserte Wirkung bei der Behandlung von Reizungen und/oder Entzündungen des Auges hat und sich kostengünstiger herstellen lässt als herkömmliche hyaluronsäurehaltige ophthalmologische Zusammensetzungen.

Diese Aufgabe wird durch die ophthalmologische Zusammensetzung gemäß den Merkmalen des Patentanspruchs 1 gelöst. Die abhängigen Patentansprüche stellen dabei vorteilhafte Weiterbildungen dar.

Die vorliegende Erfindung betrifft somit eine ophthalmologische Zusammensetzung, enthaltend oder bestehend aus
- 0,10 bis 0,45 Gew.-% Hyaluronsäure und/oder einem ophthalmologisch akzeptablen Salz der Hyaluronsäure,
- 0,60 bis 5,00 Gew.-% Ectoin oder einem ophthalmologisch akzeptablem Ectoinderivat, sowie
- ad 100 Gew.-% Wasser,
wobei die Zusammensetzung keinen weiteren pharmazeutisch wirksamen Inhaltsstoff umfasst.

Die erfindungsgemäße Lösung befeuchtet Hornhaut und Bindehaut und schützt vor übermäßiger Verdunstung der Tränen. Diese Stabilisierung des Tränenfilms lindert Augenreizungen, die mit entzündlichen Symptomen verbunden sind, oder die durch Allergien hervorgerufen werden. Das brennende und juckende Gefühl der Augen verschwindet.

Natriumhyaluronat ist eine natürliche Substanz, die im Auge, aber auch in anderen Körperteilen zu finden ist. Sie sorgt dafür, dass ein gleichmäßiger, stabiler und besonders lang haftender Feuchtigkeitsfilm auf der Oberfläche des Auges gebildet wird, der nicht rasch abgespült werden kann.

Ectoin erhöht die Wasserbindung an den Zellen der Augenoberfläche und bildet damit eine physiologische Barriere auf der Bindehaut, z.B. für allergen wirkende Stoffe. Gleichzeitig stabilisiert Ectoin den fettlöslichen Anteil des Tränenfilms, der vor einer übermäßigen Verdunstung der Tränenflüssigkeit schützt.

Überraschenderweise konnte bei der erfindungsgemäßen Zusammensetzung eine synergistische Wirkung von Hyaluronsäure bzw. hiervon abgeleiteten Derivaten sowie Ectoin auf die Viskosität festgestellt werden. Werden diese Komponenten in den erfindungsgemäßen Konzentrationen eingesetzt, so wird beobachtet, dass die Zusammensetzung eine höhere Viskosität als die Einzellösungen, die lediglich einen der genannten Bestandteile (Hyaluronsäure bzw. Ectoin) enthalten, aufweist. Die Viskosität der erfindungsgemäßen Zusammensetzung ist sogar höher als die Summe der Viskositäten der Einzellösungen.

Gleiches gilt für die Wasserbindungskapazität: Es hat sich überraschend gezeigt, dass es neben der synergistischen Erhöhung der Viskosität auch zu einer synergistischen Erhöhung der Wasserbindungskapazität durch Kombination beider Substanzen kommt. Dies ermöglicht eine bessere und intensivere Befeuchtung des Auges und Stabilisierung des Tränenfilmes gegenüber vergleichbaren Augentropfenformulierungen. Mit minimalem Rohstoffeinsatz kann ein maximaler Effekt durch das synergistische Zusammenwirken erzielt werden.

Diese Befunde haben auch eine positive Auswirkung auf die Produktionskosten der Zusammensetzung. Sowohl Hyaluronsäure als auch Ectoin sind teure Rohstoffe. Die synergistische Viskositäts- und Wasserbindungskapazitätserhöhung erweist sich somit als sehr vorteilhaft für eine Rohstoff- und Kosteneinsparung. Durch den synergistischen Effekt, kann eine ausgewählte Viskosität und Wasserbindungskapazität mit geringerem Rohstoff-Einsatz hergestellt werden als bei ophthalmologische Zusammensetzung aus dem Stand der Technik, wodurch Kosten eingespart werden können

Die erfindungsgemäße Zusammensetzung, die sowohl Ectoin und/oder ein Ectoinderivat als auch Hyaluronsäure und/oder ein Salz der Hyaluronsäure in den genannten Konzentrationen beinhaltet, haftet bei Applikation besser an der Hornhaut bzw. der Augenoberfläche des Auges als herkömmliche ophthalmologische Lösungen. Somit ist eine Aufrechterhaltung eines schützenden Films auf der Augenoberfläche gewährleistet, das Auge ist effektiver vor äußeren Einflüssen geschützt.

Im Zusammenwirken von Natriumhyaluronat und Ectoin wird das Auge außerdem mit einem intensiven, lang haftenden Feuchtigkeitsfilm versorgt und vor der Verdunstung der Tränen geschützt. Damit werden Umgebungs- und Trockenheits-bedingte Reizungen, die zu entzündlichen Symptomen führen, genauso gelindert wie die typischen Symptome Juckreiz und Brennen, die bei allergischen Reaktionen auftreten.

Die erfindungsgemäße Zusammensetzung eignet sich insbesondere zur Behandlung oder Prophylaxe des trockenen Auges (Sicca-Syndrom), zur Behandlung oder Prophylaxe der Bindehautentzündung (Conjunctivitis) und/oder zur Behandlung oder Prophylaxe von allergischen Reaktionen des Auges, wie z.B. Heuschnupfen.

Darüber hinaus schützt die erfindungsgemäße Zusammensetzung das Auge vor frühzeitigen Zellschäden. Aus der Literatur ist bereits bekannt, dass es unter hyperosmolaren Bedingungen, also osmotischem Stress, sehr schnell zur Bildung reaktiver Sauerstoffspezies (ROS) kommt. Dies wurde beispielsweise an primären humanen Cornea Epithelzellen gezeigt (Ruzhi Deng, Xia Hua, Jin Li, Wei Chi, Zongduan Zhang, Fan Lu, Lili Zhang, Stephen C. Pflugfelder, and De-Quan Li, Oxidative Stress Markers Induced by Hyperosmolarity in Primary Human Corneal Epithelial Cells, PLoS One. 2015; 10(5): e0126561). Diese reaktiven Moleküle, die unabhängig vom Vorliegen einer Entzündung, als Reaktion auf den osmotischen Stress gebildet werden, führen zu Zellschäden (Lipidperoxidation, oxidative Veränderung von Proteinen, und oxidative DNA Schäden) bis hin zur Apoptose.

Die Kombination aus Hyaluronsäure und Ectoin in Form viskoser Augentropfen wirkt auf verschiedenen Ebenen sowohl der Entstehung solcher reaktiven Moleküle entgegen, als auch Schaden begrenzend, wenn ROS entstehen.

Einerseits hat Ectoin eine Protein stabilisierende Funktion. Dadurch kann Ectoin auch Antioxidantien im Tränenfilm, wie z.B. die Cu-Zn-SOD stabilisieren. Andererseits ist Ectoin eine stark kosmotrophe Substanz die eine starke Interaktion mit Wasser zeigt. Es fördert die Bildung von Wassermolekülen in Clustern und erhöht die Oberflächenspannung des Wassers, was einer Verdunstung entgegenwirkt und entsprechend den osmotischen Stress vermindert oder verhindert. Da Ectoin aber selbst osmotisch aktiv ist, der osmotische Stress am Auge aber vermindert werden soll, ist ein minimaler Einsatz von Ectoin erwünscht. Durch den synergistischen Effekt in Kombination mit Hyaluronsäure ist gewährleistet, dass die vorteilhaften Eigenschaften von Ectoin auf Wasserbindungskapazität und Viskosität bei geringer Ectoin Konzentration eine maximale Wirkung erzielen. Zuletzt vermittelt die synergistische Viskositätserhöhung durch die Kombination einer Ectoin- und einer Hyaluronsäurekomponente eine längere Verweilzeit und somit einen verlängerten Schutz, da eine verlängerte Befeuchtung an der Augenoberfläche den osmotischen Stress und damit die Entstehung von ROS vermindert.

Weiter bevorzugt hierbei ist, dass der Gehalt an Hyaluronsäure und/oder einem ophthalmologisch akzeptablen Salz der Hyaluronsäure von 0,10 bis 0,45 Gew.-%, weiter bevorzugt von 0,125 bis 0,45 Gew.-%, besonders bevorzugt von 0,15 bis 0,25 Gew.-% , insbesondere 0,15 bis 0,20 Gew.-% beträgt.

Alternativ oder additiv ist es ebenso bevorzugt, wenn der Gehalt an Ectoin oder einem ophthalmologisch akzeptablem Ectoinderivat von 0,75 bis 3,00 Gew.-%, bevorzugt von 1,00 bis 3,00 Gew.-%, beträgt.

Das ophthalmologisch akzeptable Salz der Hyaluronsäure ist dabei bevorzugt ausgewählt aus der Gruppe bestehend aus Natrium-Hyaluronat, Kalium-Hyaluronat sowie Mischungen oder Kombinationen hiervon.

Das Ectoin ist hierbei insbesondere L-Ectoin ((S)-2-Methyl-1,4,5,6-tetrahydropyrimidin- 4-carbonsäure). Bevorzugte Ectoinderivate sind dabei ausgewählt aus der Gruppe bestehen aus Hydroxyectoin ((4S,5S)-5-Hydroxy-2-methyl-1,4,5,6-tetrahydropyrimidin-4-carbonsäure); Salzen, z. B. Natrium- oder Kaliumsalzen des Ectoins; Estern, die durch Umsetzung der 4-Carboxygruppe mit Alkoholen, insbesondere geradkettigen oder verzweigten ein- oder zweiwertigen Alkoholen mit 1 bis 20 Kohlenstoffatomen, und/oder der 5-Hydroxygruppe mit Carbonsäuren, insbesondere geradkettigen oder verzweigtkettigen ein- oder zweiwertigen Alkylcarbonsäuren mit 2 bis 20 Kohlenstoffatomen, z. B. Alkylmonocarbonsäuren, erhalten werden können sowie Säureadditionssalze mit anorganischen oder organischen Säuren.

Hierbei ist es insbesondere bevorzugt, wenn die Alkylreste der Alkohole oder Carbonsäuren jeweils bis zu 10 Kohlenstoffatome, insbesondere bis zu 5 Kohlenstoffatome aufweisen.

Gemäß einer besonders bevorzugten Ausführungsform ist die erfindungsgemäße Zusammensetzung frei von Konservierungsmitteln. Dabei wird unter Konservierungsmittel erfindungsgemäß jegliche Substanz verstanden, die als ophthalmologisches Konservierungsmittel eingesetzt werden kann, wie z. B. die weiter unten stehend aufgelisteten Konservierungsmittel.

Konservierungsmittel können den präcornealen Tränenfilm schädigen und zu einer Reduzierung der Anzahl der Mikrovilli und Mikroplicae der oberflächlichen Hornhautepitelzellen führen, was eine Reizung und/oder Entzündung des Auges zur Folge hat. Durch den Verzicht auf Konservierungsmittel in der erfindungsgemäßen Lösung kann somit eine solche Reizung und/oder Entzündung vermieden werden.

In einer alternativen bevorzugten Ausführungsform kann die erfindungsgemäße Zusammensetzung jedoch ebenso ein oder mehrere Konservierungsmittel enthalten, insbesondere ophthalmologisch verwendbare bzw. zugelassene Konservierungsmitteln. Bevorzugt sind diese Konservierungsmittel ausgewählt aus der Gruppe bestehend aus quaternären Ammoniumverbindungen, wie zum Beispiel Benzalkoniumchlorid, Cetrimid oder Polyquaternium 1; Quecksilberverbindungen, wie zum Beispiel Thiomersal oder Phenylmerkuriat; Alkoholen, wie zum Beispiel Chlorbutanol; Carbonsäuren, wie zum Beispiel Sorbinsäure; Phenole, wie zum Beispiel Parabene; Amidine, wie zum Beispiel Chlorhexidin; EDTA, insbesondere das Dinatriumsalz von EDTA; Natriumhydroxymethylglucinat; Natriumperborat; Phosphonsäure; Polydroniumchlorid; Natriumchlorit sowie Mischungen oder Kombinationen hiervon. Bevorzugt ist allerdings, dass die Zusammensetzung frei von Konservierungsmitteln, insbesondere frei von den zuvor genannten Konservierungsmitteln ist.

Weiter vorteilhaft ist, dass die ophthalmologische Zusammensetzung mindestens ein Puffersystem enthält, bevorzugt ein Puffersystem ausgewählt aus der Gruppe bestehend aus Boratpuffer, Citratpuffer, Phosphatpuffer, Tris-Puffer, Trometamol/Maleinsäure sowie Mischungen oder Kombinationen hiervon. Insbesondere ist es hierbei von Vorteil, dass die ophthalmologische Zusammensetzung aus Ectoin bzw. einem Ectoinderivat, Hyaluronsäure bzw. einem Hyaluronsäurederivat, einem Puffersystem und Wasser besteht.

Ebenso ist jedoch die Möglichkeit gegeben, dass die ophthalmologische Zusammensetzung kein Puffersystem enthält.

Eine besonders bevorzugte Ausführungsform sieht vor, dass die ophthalmologische Zusammensetzung frei von Phosphat ist. Phosphatfrei bedeutet im Sinne der vorliegenden Erfindung, dass Phosphationen wenn überhaupt nur unter Nachweisgrenze gängiger analytischer Methoden enthalten sind.

Erfindungsgemäß wird unter Phosphat jegliche Art von Phosphat verstanden, also z.B. auch Hydrogenphosphat, Dihydrogenphosphat, Diphosphat, Triphosphat, Polyphosphat und Cyclophosphat. Dies hat im Rahmen dieser bevorzugten Ausführungsform auch zur Folge, dass die Lösung keinen Phosphatpuffer enthalten darf.

Bei Langzeitanwendungen von phosphathaltigen Lösungen im Auge kann es zu einer Trübung der Hornhaut durch Ein- und/oder Ablagerungen von schwer löslichen Phosphaten wie z.B. Calciumphosphat, die sich in oder auf der Hornhaut sowie der Bindehaut des Auges einlagern oder ablagern, kommen. Diese Degeneration der Hornhaut des Auges bezeichnet man auch als Hornhautbanddegeneration oder bandförmige Keratopathie. Bereits geringfügige Ein- und/oder Ablagerungen von schwer löslichen Phosphaten in bzw. auf der Hornhaut des Auges führen zu einer massiv erhöhten Blendempfindlichkeit, die auf eine an den Ab- oder Einlagerungen von schwerlöslichen Phosphaten erfolgende Lichtstreuung zurückzuführen ist. Insbesondere wird das Sehvermögen in der Nacht hierdurch stark beeinträchtigt. Durch den bevorzugten völligen Verzicht auf Phosphat in der erfindungsgemäßen Lösung kann somit die Bildung von schwerlöslichen Phosphaten und die damit einhergehende Verschlechterung des Sehvermögens aufgrund einer Trübung der Hornhaut vermieden werden.

In einer bevorzugten Ausführungsform, insbesondere bei Vorhandensein eines Puffersystems, wird die Osmolalität (bzw. die Tonizität) der Lösung auf 100-1000 mOsm/kg, bevorzugt 200-500 mOsm/kg, besonders bevorzugt 220-350 mOsm/kg eingestell.

In einer besonders bevorzugten Ausführungsform ist der Puffer Boratpuffer.

Es ist hierbei besonders bevorzugt, dass die erfindungsgemäße Lösung Ectoin, Natriumhyaluronat, Boratpuffer und Wasserenthält oder hieraus besteht. Alternativ ist es ebenfalls bevorzugt, dass die erfindungsgemäße Lösung Ectoin, Natriumhyaluronat, Boratpuffer, einem Konservierungsmittel und Wasser enthält oder besteht.

Weiterhin ist bevorzugt, dass der Boratpuffer Borsäure und Borax enthält oder aus Borsäure und Borax besteht.

In einer weiteren bevorzugten Ausführungsform der erfindungsgemäßen Lösung beträgt der Anteil an Borsäure in der Lösung 2,5 mg/ml bis 10 mg/ml, bevorzugt 7,2 mg/ml bis 8,8 mg/ml, besonders bevorzugt 7,7 mg/ml bis 7,9 mg/ml, insbesondere 7,80 mg/ml bis 7,82 mg/ml.

In einer weiteren bevorzugten Ausführungsform der erfindungsgemäßen Lösung beträgt der Anteil an Borax in der Lösung 0,1 mg/ml bis 1 mg/ml, bevorzugt 0,3 mg/ml bis 0,7 mg/ml, besonders bevorzugt 0,4 mg/ml bis 0,5 mg/ml, insbesondere 0,41 mg/ml bis 0,43 mg/ml.

Bevorzugte pH-Werte der ophthalmologischen Zusammensetzung liegen dabei im Bereich von 5 bis 9, bevorzugt von 6 bis 8, insbesondere von 6,8 bis 7,8.

Die ophthalmologische Zusammensetzung gemäß der vorliegenden Erfindung weist bevorzugt eine kinematische Viskosität, gemessen mittels Kapillarviskosimetrie wie in PhEur 7.2, Allgemeine Methoden 2.2.8. beschrieben von 10 bis 500 mm²/s, bevorzugt 30 bis 300 mm²/s, besonders bevorzugt von 50 bis 250 mm²/s, auf.

Insbesondere ist die erfindungsgemäße Zusammensetzung steril.

Bevorzugt ist die ophthalmologische Zusammensetzung gemäß der vorliegenden Erfindung in Form von Augentropfen oder eines Augengels ausgebildet.

Die ophthalmologische Zusammensetzung kann 1 bis 10 mal täglich, bevorzugt 2 bis 6 mal täglich im Auge appliziert werden. Insbesondere wird die ophthalmologische Zusammensetzung durch Eintropfen in das Auge appliziert.

In einer Ausführungsform weist die ophthalmologische Zusammensetzung die folgende Formulierung auf:

| | |
|---|---|
| Natriumhyaluronat | 0,55 bis 5 mg |
| Ectoin | 0,50 bis 3 mg |
| Borsäure | 2,5 bis 10 mg |
| Borax | 0,1 bis 1 mg |
| Wasser ad | 1 ml |

Die erfindungsgemäße Zusammensetzung mit dieser Formulierung hat eine Dichte von ca. 1,0068 g/cm³. Die Lösung befeuchtet Hornhaut und Bindehaut und schützt vor übermäßiger Verdunstung der Tränen. Sie kann zur Vorbeugung und Behandlung einer Reizung und/oder Entzündung des Auges verwendet werden, wobei die Reizung und/oder Entzündung des Auges durch eine unzureichende Benetzung des Auges mit einem Tränenfilm und/oder durch eine Überempfindlichkeit und/oder Allergie hervorgerufen wird. Das brennende und juckende Gefühl der Augen, welches durch Reizung und/oder Entzündung verursacht wird, verschwindet.

Weiterhin ist bevorzugt, dass die Reizung und/oder Entzündung des Auges durch eine unzureichende Benetzung des Auges mit einem Tränenfilm und/oder durch eine Überempfindlichkeit und/oder Allergie hervorgerufen wird.

Die vorliegende Erfindung wird anhand der nachfolgenden Ausführungen näher beschrieben, ohne die vorliegende Erfindung auf die speziell dargestellten Versuche zu beschränken.

### Versuchsbeschreibung:

Es wurden insgesamt 12 Lösungen mit unterschiedlichen Konzentrationen an Hyaluronsäure und Ectoin in einer Rezepturgrundlage hergestellt:
0. Herstellung Rezepturgrundlage ohne Hyaluronsäure und ohne Ectoin:
   Zunächst werden 781 mg Borsäure und 42 mg Borax nacheinander in etwa 80 ml destilliertem Wasser gelöst. Nachdem die Rohstoffe vollständig gelöst sind, wird auf 100 ml mit destilliertem Wasser aufgefüllt.
1. Herstellung Rezepturgrundlage ohne Hyaluronsäure und mit 1 % Ectoin:
   Es werden 1 g Ectoin, 781 mg Borsäure und 42 mg Borax nacheinander in etwa 965 ml destilliertem Wasser gelöst. Nachdem die Rohstoffe vollständig gelöst sind, wird auf 100 ml mit destilliertem Wasser aufgefüllt.
2. Herstellung Rezepturgrundlage ohne Hyaluronsäure und mit 2 % Ectoin:
   Es werden 2 g Ectoin, 781 mg Borsäure und 42 mg Borax nacheinander in etwa 95 ml destilliertem Wasser gelöst. Nachdem die Rohstoffe vollständig gelöst sind, wird auf 100 ml mit destilliertem Wasser aufgefüllt.
3. Herstellung Rezepturgrundlage ohne Hyaluronsäure und mit 3 % Ectoin:
   Es werden 3 g Ectoin, 781 mg Borsäure und 42 mg Borax nacheinander in etwa 95 ml destilliertem Wasser gelöst. Nachdem die Rohstoffe vollständig gelöst sind, wird auf 100 ml mit destilliertem Wasser aufgefüllt.
4. Herstellung Rezepturgrundlage mi 0,1 % Hayluronsäure und ohne Ectoin:
   Zunächst werden in einem Becherglas etwa 45 ml destilliertes Wasser vorgelegt und 100 mg Hyaluronsäure darin gelöst (Lösung 1). In einem 2. Becherglas werden 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
5. Herstellung Rezepturgrundlage mit 0,2 % Hyaluronsäure und ohne Ectoin:
   Zunächst werden in einem Becherglas etwa 45 ml destilliertes Wasser vorgelegt und 200 mg Hyaluronsäure darin gelöst (Lösung 1). In einem 2. Becherglas werden 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
6. Herstellung Rezepturgrundlage mit 0,1 % Hyaluronsäure und 1 % Ectoin:
   Zunächst werden in einem Becherglas etwa 45 ml destilliertes Wasser vorgelegt und 100 mg Hyaluronsäure darin gelöst (Lösung 1). In einem 2. Becherglas werden 1 g Ectoin, 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
7. Herstellung Rezepturgrundlage mit 0,1 % Hyaluronsäure und 2 % Ectoin:
   Zunächst werden in einem Becherglas etwa 45 ml destilliertes Wasser vorgelegt und 100 mg Hyaluronsäure darin gelöst (Lösung 1). In einem 2. Becherglas werden 2 g Ectoin, 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
8. Herstellung Rezepturgrundlage mit 0,1 % Hyaluronsäure und 3 % Ectoin:
   Zunächst werden in einem Becherglas etwa 45 ml destilliertes Wasser vorgelegt und 100 mg Hyaluronsäure darin gelöst (Lösung 1). In einem 2. Becherglas werden 3 g Ectoin, 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
9. Herstellung Rezepturgrundlage mit 0,2 % Hyaluronsäure und 1 % Ectoin:
   Zunächst werden in einem Becherglas etwa 45 ml destilliertes Wasser vorgelegt und 200 mg Hyaluronsäure darin gelöst (Lösung 1). In einem 2. Becherglas werden 1 g Ectoin, 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
10. Herstellung Rezepturgrundlage mit 0,2 % Hyaluronsäure und 2 % Ectoin:
   Zunächst werden in einem Becherglas etwa 45 ml destilliertes Wasser vorgelegt und 200 mg Hyaluronsäure darin gelöst (Lösung 1). In einem 2. Becherglas werden 2 g Ectoin, 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.
11. Herstellung Rezepturgrundlage mit 0,2 % Hyaluronsäure und 3 % Ectoin:
   Zunächst werden in einem Becherglas etwa 45 ml destilliertes Wasser vorgelegt und 200 mg Hyaluronsäure darin gelöst (Lösung 1). In einem 2. Becherglas werden 3 g Ectoin, 781 mg Borsäure und 42 mg Borax nacheinander in etwa 45 ml destilliertem Wasser gelöst (Lösung 2). Nachdem alle Rohstoffe vollständig gelöst sind, wird Lösung 2 langsam und unter Rühren zu Lösung 1 gegeben. Danach wird auf 100 ml mit destilliertem Wasser aufgefüllt.

Die wie zuvor hergestellten Lösungen wurden auf ihre Viskosität hin untersucht.

Die Viskositätsmessungen wurden mit einem Ubbelohde-Viskosimeter 501 20/II wie in PhEur 7.2, Allgemeine Methoden 2.2.8 beschrieben durchgeführt.

Für die einzelnen Zusammensetzungen wurden dabei die folgenden Viskositätswerte erhalten, die in der nachfolgenden Tabelle wiedergegeben sind:

| | | Viskosität, Messung mit Kapillare II (mm²/s) |
|---|---|---|
| 0^{∗} | Rezepturgrundlage ohne HA und ohne Ectoin | 1,2 |
| 1^{∗} | Rezepturgrundlage ohne HA und 1% Ectoin | 1,2 |
| 2^{∗} | Rezepturgrundlage ohne HA und 2% Ectoin | 1,3 |
| 3^{∗} | Rezepturgrundlage ohne HA und 3% Ectoin | 5,8 |
| 4^{∗} | Rezepturgrundlage ohne Ectoin und 0,1 % HA | 18,3 |
| 5^{∗} | Rezepturgrundlage ohne Ectoin und 0,2 % HA | 93,0 |
| 6 | Rezepturgrundlage 0,1 % HA und 1% Ectoin | 20,8 |
| 7 | Rezepturgrundlage 0,1 % HA und 2 % Ectoin | 21,4 |
| 8 | Rezepturgrundlage 0,1 % HA und 3 % Ectoin | 22,4 |
| 9 | Rezepturgrundlage 0,2 % HA und 1% Ectoin | 144,1 |
| 10 | Rezepturgrundlage 0,2 % HA und 2 % Ectoin | 146,7 |
| 11 | Rezepturgrundlage 0,2 % HA und 3 % Ectoin | 158,6 |

| | | |
|---|---|---|
| ^{∗}Vergleichsversuche | | |

Wie aus den erfindungsgemäßen Versuchen 6 bis 8 bzw. 9 bis 11 ersichtlich ist, findet eine deutliche, synergistische Beeinflussung der Viskosität bei Anwesenheit von Hyaluronsäure und Ectoin statt, wobei die Viskositäten bei diesen kombinatorischen Versuchen höher ausfallen, als die Summe der einzelnen Messwerte in den Einzelversuchen (Versuche 1 bis 3 bzw. Versuche 4 bis 5).

Außerdem wurde die Wasserbindungskapazität von sieben verschiedenen Mischungen (Nr. 12-18) aus Hyaluronsäure und Ectoin mit unterschiedlichen Gewichtsverhältnissen HA:Ectoin ermittelt. Dazu wurden zwei verschiedene Methoden angewandt.

Einerseits wurde die Wasserbindungskapazität mit Hilfe einer gravimetrischen Methode bestimmt. Hierbei wurden die in der nachfolgenden Tabelle angegebenen Anteile m_{HA} und m_{Ec} an Hyaluronsäure und Ectoin in ein Eppendorfgefäß vorgelegt, wobei sich die Anteile auf ein Gesamtgewicht (Hyaluronsäure+Ectoin) von 100 mg beziehen. Die Gesamtmasse des gefüllten Eppendorfgefäßes wurde notiert. Anschließend wurde so lange Wasser zugetropft, bis ein geringer Wasserüberschuss vorlag und eine klare Lösung entstanden war. Diese Lösung wurde einer Zentrifugation bei 200 rpm unterzogen. Dabei wurde das überstehende Wasser abgenommen und das Gesamtgewicht des Eppendorfgefäßes wurde erneut bestimmt. Die Wasserbindungskapazität wurde dann als Differenz des Gesamtgewichts nach der Zentrifugation und des anfänglich notierten Gesamtgewichts errechnet.

Die nachfolgende Tabelle zeigt die Ergebnisse der gravimetrischen Bestimmung der prozentualen Wasserbindungskapazität (WBK). Außerdem zeigt die Tabelle die Werte für die Wasserbindungskapazität, die bei einem rein additiven Effekt aufgrund der WBK für pure Hyaluronsäure (Versuch 12) und der WBK für pures Ectoin (Versuch 18) zu erwarten gewesen wäre. Die Abweichung der gemessenen WKB von der berechneten theoretischen WBK bildet die synergistische Wirkung ab.

| Mischung | m_{HA} in % | m_{Ec} in % | WBK (gravimetrisch) in % | WBK berechnet in % | Synergistische Wirkung in % |
|---|---|---|---|---|---|
| 12 | 100 | 0 | 32,00 | - | - |
| 13 | 20 | 80 | 45,91 | 43,0 | 6,8 |
| 14 | 40 | 60 | 70,04 | 54,0 | 29,7 |
| 15 | 50 | 50 | 74,00 | 59,5 | 24,4 |
| 16 | 60 | 40 | 74,80 | 65,0 | 15,1 |
| 17 | 80 | 20 | 79,20 | 76,0 | 4,2 |
| 18 | 0 | 100 | 87,00 | - | - |

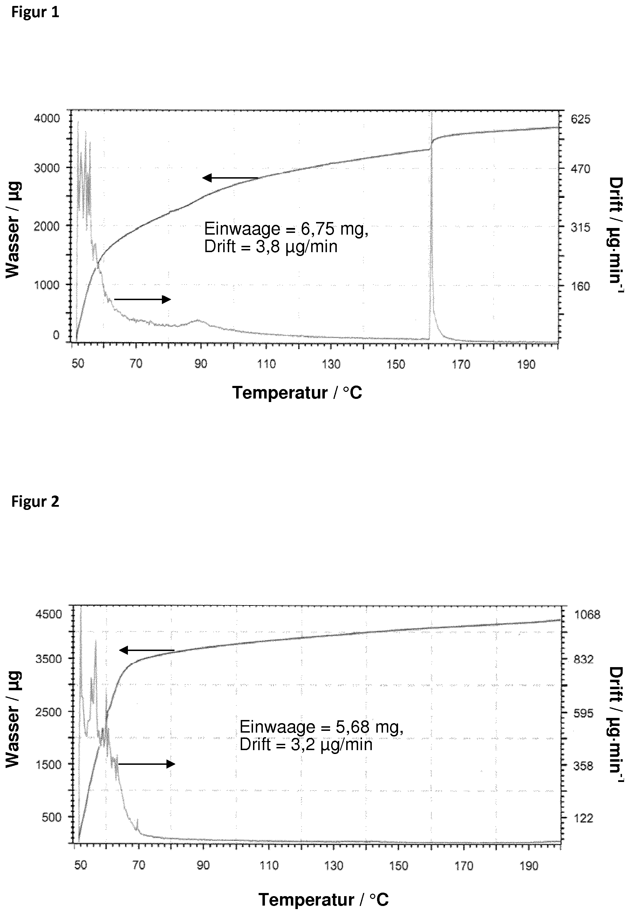

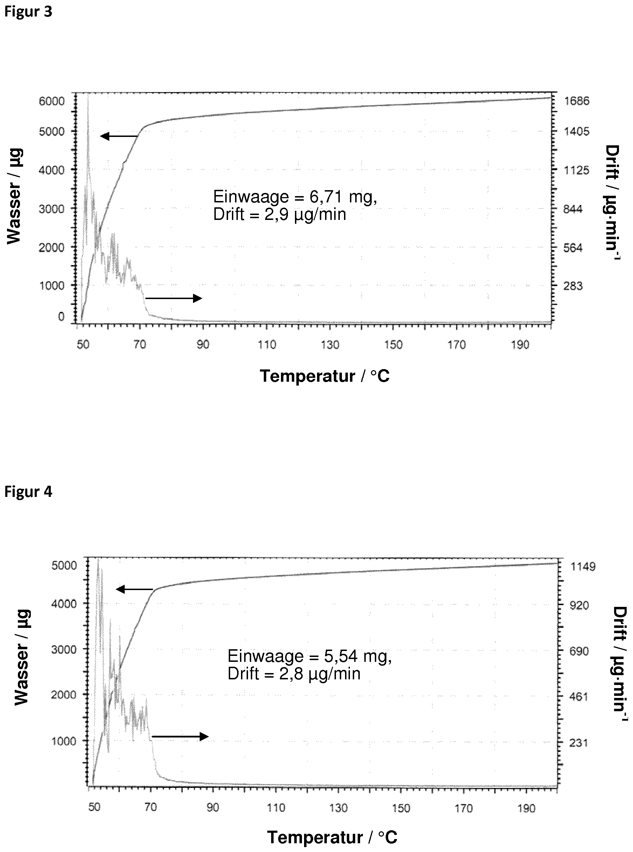

Andererseits wurde die Wasserbindungskapazität mittels der indirekten Karl-Fischer Titration mit Ofentechnologie bestimmt. Hierzu wurde aus dem Eppendorfgefäß nach der Zentrifugation und dem Abziehen des überstehenden Wassers in der gravimetrischen Methode eine Probe von 5 -7 mg entnommen. Diese Probe wurde auf eine Anfangstemperatur von 50°C erhitzt und anschließend in einem verschlossenen Gefäß mit einer Heizrate von 2°C/min auf 200°C erhitzt. Durch eine Gasspülung wurde das dabei verdampfende Wasser über eine Hohlnadel in eine Titrierzelle geleitet. Das dort gesammelte Wasser reagierte mit einer Karl Fischer-Lösung und über den Endpunkt der Titrierkurve wurden der Wassergehalt der Probe und die Wasserbindungskapazität berechnet. Die Figuren 1-4 zeigen die Thermogramme, die bei der oben beschriebenen Karl Fischer Analyse der Mischungen 13, 14, 16 und 17 aufgezeichnet wurden: Fig. 1 zeigt die Messkurven, die bei der Analyse von Mischung 13 erhalten wurden. Fig.2 zeigt die Thermogramme der Analyse von Mischung 14. Fig.3 zeigt die bei der Analyse der Mischung 16 erhaltenen Thermogramme und Fig.4 die Ergebnisse der Analyse der Mischung 17.

Aus diesen Figuren kann die Einwaage der Probe sowie die detektierte Masse an Wasser bei 200°C abgelesen werden. Zur Korrektur der ermittelten Masse an Wasser muss außerdem die zeitliche Drift der Messapparatur hinzugezogen werden.

Die nachfolgende Tabelle fasst die Ergebnisse der Bestimmung der prozentualen Wasserbindungskapazität (WBK) nach der indirekten Karl-Fischer Methode mit Ofentechnologie zusammen. Außerdem zeigt die Tabelle die Werte für die Wasserbindungskapazität, die bei einem rein additiven Effekt aufgrund der WBK für pure Hyaluronsäure (Versuch 12) und der WBK für pures Ectoin (Versuch 18) zu erwarten gewesen wäre. Die Abweichung der gemessenen WKB von dieser theoretisch errechneten WBK bildet auch hier ein Maß für die synergistische Wirkung.

| Mischung | m_{HA} in % | m_{Ec} in % | WBK (nach Karl Fischer) in % | WBK berechnet in % | Synergistische Wirkung in % |
|---|---|---|---|---|---|
| 12 | 100 | 0 | 31,80 | - | - |
| 13 | 20 | 80 | 50,60 | 44,2 | 14,5 |
| 14 | 40 | 60 | 64,30 | 56,6 | 13,6 |
| 15 | 50 | 50 | 80,20 | 62,8 | 27,7 |
| 16 | 60 | 40 | 83,65 | 69,0 | 21,2 |
| 17 | 80 | 20 | 83,95 | 81,4 | 3,1 |
| 18 | 0 | 100 | 93,80 | - | - |

Die Ergebnisse der Bestimmung der WBK belegen, dass es - unabhängig von der Messmethode - einen synergistischen Effekt von Hyaluronsäure und Ectoin auf die Wasserbindungskapazität gibt. Bei einem rein additiven Effekt wäre bei der Mischung 15 beispielsweise ein Wert von 62,8 % (= 0,5 · 32,00 +0,5 · 87,00) für Wasserbindungskapazität mittels Karl-Fischer Titration zu erwarten gewesen, gemessen wurde aber ein Wert von 80,2%. Das Gleiche gilt für die gravimetrische Bestimmung: Bei einem rein additivem Effekt wäre für Mischung 15 eine Wasserbindungskapazität von 59,5% (= 0,5 · 31,80 +0,5 · 93,80) zu erwarten gewesen, gemessen wurde aber eine WBK von 74,0 %.

## Patentansprüche

1. Ophthalmologische Zusammensetzung, enthaltend oder bestehend aus
0,10 bis 0,45 Gew.-% Hyaluronsäure und/oder einem ophthalmologisch akzeptablen Salz der Hyaluronsäure,
0,60 bis 5,00 Gew.-% Ectoin oder einem ophthalmologisch akzeptablem Ectoinderivat, sowie
ad 100 Gew.-% Wasser,
wobei die Zusammensetzung keinen weiteren pharmazeutisch wirksamen Inhaltsstoff umfasst.

2. Ophthalmologische Zusammensetzung nach Anspruch 1, **dadurch gekennzeichnet, dass** der Gehalt an
Hyaluronsäure und/oder einem ophthalmologisch akzeptablen Salz der Hyaluronsäure von 0,125 bis 0,45 Gew.-%, bevorzugt von 0,15 bis 0,25 Gew.-%, insbesondere 0,15 bis 0,20 Gew.-% und/oder
Ectoin oder einem ophthalmologisch akzeptablem Ectoinderivat von 0,75 bis 3,00 Gew.-%, bevorzugt von 1,00 bis 3,00 Gew.-%, beträgt.

3. Ophthalmologische Zusammensetzung nach einem der vorhergehenden Ansprüche, **dadurch gekennzeichnet, dass** das ophthalmologisch akzeptable Salz der Hyaluronsäure ausgewählt ist aus der Gruppe bestehend aus Natrium-Hyaluronat, Kalium-Hyaluronat sowie Mischungen oder Kombinationen hiervon.

4. Ophthalmologische Zusammensetzung nach einem der vorhergehenden Ansprüche, **dadurch gekennzeichnet, dass** das Ectoin L-Ectoin ((S)-2-Methyl-1 ,4,5,6-tetrahydropyrimidin- 4-carbonsäure) ist und/oder das Ectoinderivat ausgewählt ist aus der Gruppe bestehend aus Hydroxyectoin ((4S,5S)-5-Hydroxy-2-methyl-1,4,5,6-tetrahydropyrimidin-4-carbonsäure); Salzen, z. B. Natrium- oder Kaliumsalzen des Ectoins; Estern, die durch Umsetzung der 4-Carboxygruppe mit Alkoholen, insbesondere geradkettigen oder verzweigten ein- oder zweiwertigen Alkoholen mit 1 bis 20 Kohlenstoffatomen, und/oder der 5-Hydroxygruppe mit Carbonsäuren, insbesondere geradkettigen oder verzweigtkettigen ein- oder zweiwertigen Alkylcarbonsäuren mit 2 bis 20 Kohlenstoffatomen, z. B. Alkylmonocarbonsäuren, erhalten werden können sowie Säureadditionssalze mit anorganischen oder organischen Säuren.

5. Ophthalmologische Zusammensetzung nach vorhergehendem Anspruch, **dadurch gekennzeichnet, dass** die Alkylreste der Alkohole oder Carbonsäuren jeweils bis zu 10 Kohlenstoffatome, insbesondere bis zu 5 Kohlenstoffatome aufweisen.

6. Ophthalmologische Zusammensetzung nach einem der vorhergehenden Ansprüche, **dadurch gekennzeichnet, dass** sie frei von Konservierungsmitteln ist,
oder
mindestens ein Konservierungsmittel enthält, insbesondere ausgewählt aus der Gruppe bestehend aus quaternären Ammoniumverbindungen, wie zum Beispiel Benzalkoniumchlorid, Cetrimid oder Polyquaternium 1; Quecksilberverbindungen, wie zum Beispiel Thiomersal oder Phenylmerkuriat; Alkoholen, wie zum Beispiel Chlorbutanol; Carbonsäuren, wie zum Beispiel Sorbinsäure; Phenole, wie zum Beispiel Parabene; Amidine, wie zum Beispiel Chlorhexidin; EDTA, insbesondere das Dinatriumsalz von EDTA; Natriumhydroxymethylglucinat; Natriumperborat; Phosphonsäure; Polydroniumchlorid; Natriumchlorit sowie Mischungen oder Kombinationen hiervon.

7. Ophthalmologische Zusammensetzung nach einem der vorhergehenden Ansprüche, **dadurch gekennzeichnet, dass** sie mindestens ein Puffersystem enthält, bevorzugt ein Puffersystem ausgewählt aus der Gruppe bestehend aus Boratpuffer, Citratpuffer, Phosphatpuffer, Tris-Puffer, Trometamol/Maleinsäure sowie Mischungen oder Kombinationen hiervon
oder kein Puffersystem enthält.

8. Ophthalmologische Zusammensetzung nach einem der vorhergehenden Ansprüche, **dadurch gekennzeichnet, dass** sie frei von Phosphationen ist.

9. Ophthalmologische Zusammensetzung nach einem der vorhergehenden Ansprüche, **dadurch gekennzeichnet, dass** die Osmolalität der Lösung 100-1000 mOsm/kg, bevorzugt 200-500 mOsm/kg, besonders bevorzugt 220-350 mOsm/kg beträgt.

10. Ophthalmologische Zusammensetzung nach einem der vorhergehenden Ansprüche, **dadurch gekennzeichnet, dass** sie einen pH-Wert von 5 bis 9, bevorzugt von 6 bis 8, insbesondere von 6,8 bis 7,8 aufweist.

11. Ophthalmologische Zusammensetzung nach einem der vorhergehenden Ansprüche, **dadurch gekennzeichnet, dass** sie eine kinematische Viskosität, gemessen mittels Kapillarviskosimetrie gemessen gemäß PhEur 7.2. Allgemeine Methoden 2.2.8 von 10 bis 500 mm²/s, bevorzugt 30 bis 300 mm²/s, besonders bevorzugt von 50 bis 250 mm²/s aufweist.

12. Ophthalmologische Zusammensetzung nach einem der vorhergehenden Ansprüche, **dadurch gekennzeichnet, dass** die Zusammensetzung steril ist.

13. Ophthalmologische Zusammensetzung nach einem der vorhergehenden Ansprüche, **dadurch gekennzeichnet, dass** sie in Form von Augentropfen oder eines Augengels ausgebildet ist.

14. Ophthalmologische Zusammensetzung nach einem der vorhergehenden Ansprüche, zur Anwendung in der Behandlung oder Prophylaxe des trockenen Auges (Sicca-Syndrom), zur Behandlung oder Prophylaxe der Bindehautentzündung (Conjunctivitis) und/oder zur Behandlung oder Prophylaxe von allergischen Reaktionen des Auges, wie z.B. Heuschnupfen.

15. Ophthalmologische Zusammensetzung zur Anwendung gemäß vorhergehendem Anspruch, **dadurch gekennzeichnet, dass** sie ein bis 10 mal täglich, bevorzugt 2 bis 6 mal täglich im Auge appliziert wird, bevorzugt durch Eintropfen in das Auge.

## Claims

1. An ophthalmological composition comprising or consisting of
0.10 to 0.45% by weight of hyaluronic acid and/or an ophthalmologically acceptable salt of hyaluronic acid,
0.60 to 5.00% by weight of ectoin or an ophthalmologically acceptable ectoin derivative, and
ad 100% by weight water,
the composition not comprising any further pharmaceutically active ingredient.

2. The ophthalmological composition according to claim 1, **characterized in that** the content of
hyaluronic acid and/or an ophthalmologically acceptable salt of hyaluronic acid is from 0.125 to 0.45% by weight, preferably from 0.15 to 0.25% by weight, in particular 0.15 to 0.20% by weight and/or
ectoin or an ophthalmologically acceptable ectoin derivative is from 0.75 to 3.00% by weight, preferably from 1.00 to 3.00% by weight.

3. The ophthalmological composition according to any one of the preceding claims, **characterized in that** the ophthalmologically acceptable salt of hyaluronic acid is selected from the group consisting of sodium hyaluronate, potassium hyaluronate and mixtures or combinations thereof.

4. The ophthalmological composition according to any one of the preceding claims, **characterized in that** the ectoin is L-ectoin ((S)-2-methyl-1,4,5,6-tetrahydropyrimidine-4-carboxylic acid) and/or the ectoin derivative is selected from group consisting of hydroxyectoin ((4S,5S)-5-hydroxy-2-methyl-1,4,5,6-tetrahydropyrimidine-4-carboxylic acid); salts, for example, sodium or potassium salts of the ectoin; esters which can be obtained by reacting the 4-carboxy group with alcohols, in particular straight-chain or branched monovalent or bivalent alcohols having 1 to 20 carbon atoms, and/or the 5-hydroxy group with carboxylic acids, in particular straight-chain or branched monovalent or bivalent alkylcarboxylic acids having 2 to 20 carbon atoms, for example, alkyl monocarboxylic acids, and acid addition salts with inorganic or organic acids.

5. The ophthalmological composition according to the preceding claim, **characterized in that** the alkyl residuals of the alcohols or carboxylic acids each have up to 10 carbon atoms, in particular up to 5 carbon atoms.

6. The ophthalmological composition according to any one of the preceding claims, **characterized in that** it
is free from preservatives,
or
contains at least one preservative, in particular selected from the group consisting of quaternary ammonium compounds, such as benzalkonium chloride, cetrimide or polyquaternium 1; mercury compounds such as thiomersal or phenyl mercuriate; alcohols such as chlorobutanol; carboxylic acids such as sorbic acid; phenols such as parabens; amidines such as chlorhexidine; EDTA, in particular the disodium salt of EDTA; sodium hydroxymethyl glucinate; sodium perborate; phosphonic acid; polydronium chloride; sodium chlorite and mixtures or combinations thereof.

7. The ophthalmological composition according to any one of the preceding claims, **characterized in that** it
contains at least one buffer system, preferably a buffer system selected from the group consisting of borate buffer, citrate buffer, phosphate buffer, tris buffer, trometamol/maleic acid and mixtures or combinations thereof or no buffer system.

8. The ophthalmological composition according to any one of the preceding claims, **characterized in that** it is free from phosphate ions.

9. The ophthalmological composition according to any one of the preceding claims, **characterized in that** the osmolality of the solution is 100-1000 mOsm/kg, preferably 200-500 mOsm/kg, particularly preferably 220-350 mOsm/kg.

10. The ophthalmological composition according to any one of the preceding claims, **characterized in that** it has a pH of 5 to 9, preferably 6 to 8, in particular 6.8 to 7.8.

11. The ophthalmological composition according to any one of the preceding claims, **characterized in that** it has a kinematic viscosity, measured by means of capillary viscometry, measured according to PhEur 7.2. General Methods 2.2.8 from 10 to 500 mm²/s, preferably from 30 to 300 mm²/s, particularly preferably from 50 to 250 mm²/s.

12. The ophthalmological composition according to any one of the preceding claims, **characterized in that** the composition is sterile.

13. The oophthalmological composition according to any one of the preceding claims, **characterized in that** it is in the form of eye drops or an eye gel.

14. The ophthalmological composition according to any one of the preceding claims, for use in the treatment or prophylaxis of dry eyes (sicca syndrome), for the treatment or prophylaxis of conjunctivitis and/or for the treatment or prophylaxis of allergic reactions of the eye, such as hay fever.

15. The ophthalmological composition for use according to the preceding claim, **characterized in that** it is applied to the eye once to 10 times a day, preferably 2 to 6 times a day, preferably by dripping into the eye.

## Revendications

1. Composition ophtalmologique contenant ou comprenant
0,10 à 0,45 % en masse d'acide hyaluronique et/ou d'un sel ophtalmologiquement acceptable de l'acide hyaluronique,
0,60 à 5,00 % en masse d'ectoïne ou d'un dérivé d'éctoïne ophtalmologiquement acceptable, ainsi que
jusqu'à 100 % en masse d'eau,
dans laquelle la composition ne comprend pas d'autre ingrédient pharmaceutiquement actif.

2. Composition ophtalmologique selon la revendication 1, **caractérisée en ce que** la teneur
en acide hyaluronique et/ou un sel ophtalmologiquement acceptable de l'acide hyaluronique est comprise entre 0,125 et 0,45 % en masse, de préférence entre 0,15 et 0,25 % en masse, en particulier entre 0,15 et 0,20 % en masse, et/ou
en ectoïne ou un dérivé d'ectoïne ophtalmologiquement acceptable est comprise entre 0,75 et 3,00 % en masse, de préférence entre 1,00 et 3,00 % en masse.

3. Composition ophtalmologique selon l'une des revendications précédentes, **caractérisée en ce que** le sel ophtalmologiquement acceptable de l'acide hyaluronique est choisi dans le groupe constitué par l'hyaluronate de sodium, l'hyaluronate de potassium et leurs mélanges ou combinaisons.

4. Composition ophtalmique selon l'une des revendications précédentes, **caractérisée en ce que** l'ectoïne est la L-ectoïne ((S)-2-méthyl-acide-1,4,5,6-tétrahydropyrimidine-4-carboxylique) et/ou le dérivé d'ectoïne est choisi dans le groupe constitué par 1'hydroxyectoïne ((4S,5S)-5-hydroxy-2-méthyl-acide-1,4,5,6-tétrahydropyrimidine-4-carboxylique) ;
des sels, p. ex. des sels de sodium ou de potassium de l'ectoïne ; des esters qui peuvent être obtenus par réaction du groupe 4-carboxy avec des alcools, en particulier des alcools mono- ou bivalents à chaîne linéaire ou ramifiée comportant 1 à 20 atomes de carbone, et/ou du groupe 5-hydroxy avec des acides carboxyliques, en particulier des acides alkylcarboxyliques mono- ou divalents à chaîne linéaire ou ramifiée comportant 2 à 20 atomes de carbone, par exemple des acides alkylmonocarboxyliques, ainsi que des sels d'addition d'acides avec des acides inorganiques ou organiques.

5. Composition ophtalmologique selon la revendication précédente, **caractérisée en ce que** les radicaux alkyle des alcools ou des acides carboxyliques comportent chacun jusqu'à 10 atomes de carbone, en particulier jusqu'à 5 atomes de carbone.

6. Composition ophtalmologique selon l'une des revendications précédentes, **caractérisée en ce qu'**elle
est exempt de conservateurs,
ou
contient au moins un agent conservateur, en particulier choisi dans le groupe constitué par des composés d'ammonium quaternaire, comme le chlorure de benzalkonium, le cétrimide ou le polyquaternium 1; des composés de mercure, comme le thiomersal ou le phényl-mercurate ; des alcools, comme le chlorobutanol ; des acides carboxyliques, comme l'acide sorbique ; des phénols, comme les parabènes ; des amidines, comme la chlorhexidine ; l'EDTA, en particulier le sel disodique de l'EDTA; l'hydroxyméthylglucinate de sodium ; le perborate de sodium ; l'acide phosphoreux ; le chlorure de polidronium ; le chlorite de sodium et leurs mélanges ou combinaisons.

7. Composition ophtalmologique selon l'une des revendications précédentes, **caractérisée en ce qu'**elle
contient au moins un système tampon, de préférence un système tampon choisi dans le groupe constitué par un tampon borate, un tampon citrate, un tampon phosphate, un tampon Tris, le trométamol/l'acide maléique ainsi que leurs mélanges ou combinaisons ou ne contient pas de système tampon.

8. Composition ophtalmologique selon l'une des revendications précédentes, **caractérisée en ce qu'**elle est exempte d'ions phosphate.

9. Composition ophtalmologique selon l'une des revendications précédentes, **caractérisée en ce que** l'osmolalité de la solution est comprise entre 100 et 1 000 mOsm/kg, de préférence entre 200 et 500 mOsm/kg, plus préférablement entre 220 et 350 mOsm/kg.

10. Composition ophtalmologique selon l'une des revendications précédentes, **caractérisée en ce qu'**elle présente un pH de 5 à 9, de préférence de 6 à 8, en particulier de 6,8 à 7,8.

11. Composition ophtalmologique selon l'une des revendications précédentes, **caractérisée en ce qu'**elle présente une viscosité cinématique, mesurée par viscosimétrie capillaire mesurée selon Ph. Eur. 7.2. Méthodes générales 2.2.8, comprise entre 10 et 500 mm²/s, de préférence entre 30 et 300 mm²/s, plus préférablement entre 50 et 250 mm²/s.

12. Composition ophtalmologique selon l'une des revendications précédentes, **caractérisée en ce que** la composition est stérile.

13. Composition ophtalmologique selon l'une des revendications précédentes, **caractérisée en ce qu'**elle se présente sous la forme d'un collyre ou d'un gel ophtalmique.

14. Composition ophtalmologique selon l'une des revendications précédentes, destinée à être utilisée dans le traitement ou la prophylaxie de la sécheresse oculaire (syndrome de Sicca), dans le traitement ou la prophylaxie de la conjonctivite et/ou dans le traitement ou la prophylaxie des réactions allergiques de l'œil, comme le rhume des foins.

15. Composition ophtalmologique à utiliser selon la revendication précédente, **caractérisée en ce qu'**elle est appliquée dans l'œil de 1 à 10 fois par jour, de préférence de 2 à 6 fois par jour, de préférence par instillation dans l'œil.
